# EUROPÄISCHE PATENTSCHRIFT

(11) **EP 0 717 977 B1**
(45) Veröffentlichungstag und Bekanntmachung des Hinweises auf die Patenterteilung: **19.03.2003**
(21) Anmeldenummer: 95120102.9
(22) Anmeldetag: 19.12.1995
(51) Int. Cl.: A61K 6/083

(54) **Fluoridabgebende Composite-Massen**
Fluoride releasing dental composite material
Matériau composite dentaire à libération de fluorure

(30) Priorität: 19.12.1994 DE 4445266
(43) Veröffentlichungstag der Anmeldung: 26.06.1996
(73) Patentinhaber: 3M ESPE AG, 82229 Seefeld (DE)
(72) Erfinder: Nowak, Reinhold, Dr., D-82276 Adelshofen (DE); Wanek, Erich, Dr., D-86916 Kaufering (DE); Gangnus, Bernd, Dr., D-82211 Herrsching (DE)
(74) Vertreter: Abitz, Walter, Dr.-Ing.

(56) Entgegenhaltungen:
- EP-A- 0 238 025
- DE-A- 4 032 505
- US-A- 4 871 786

## Beschreibung

Die Bildung von Sekundärkaries an den Rändern von dentalen Dauerfüllungen ist ein in der Zahnmedizin seit langem bekanntes Problem. Die Tendenz zur Bildung von Sekundärkaries in dentalen Füllungsmaterialien reduziert sich nachgewiesenermaßen, wenn sie in der Lage sind, Fluorid in die umgebende Zahnhartsubstanz abzugeben. Die Ursache hierfür ist vermutlich die Bildung des kariesresistenten Fluorapatits durch Reaktion des Hydroxylapatits des Zahns mit freigesetztem Fluorid (R.S. Levine, The Action of Fluoride in Caries Prevention, British Dental Journal 140 (1976) 9-14).

Über die Frage, welche Menge an Fluorid aus einem Füllungsmaterial ausgelöst werden muß, damit dieses zuverlässig kariesinhibierend wirkt, herrscht in der Literatur keine Einigkeit (R.W. Phillips, Restorative Materials Containing Fluoride, Journal of American Dental Association 116 (1988) 762-763). Aufgrund von klinischen Erfahrungen mit verschiedenen fluoridabgebenden Füllungsmaterialien zeichnet sich jedoch ab, daß die Menge an Fluorid, die durch Glasionomerzemente freigesetzt wird, klinisch relevant die Bildung von Sekundärkaries vermindern kann (G. Wesenberg et al., J. Oral Rehabil. 7 (1980) 175-184). Weiterhin zeigt sich, daß bei sogenannten Composite-Füllungswerkstoffen, die keine oder nur sehr geringe Fluoridabgabe zeigen, eine besondere Anfälligkeit gegen Sekundärkariesbefall besteht (E.A.M. Kidd, Br. Dent. J. 144 (1978) 139-142). Durch die Schrumpfung des Kunststoffes beim Härten kann ein Randspalt entstehen, welcher die Bildung von Karies begünstigt.

Es hat daher nicht an Versuchen gefehlt, Composite-Füllungswerkstoffe bereitzustellen, die eine den Glasionomerzementen vergleichbare Fluoridionenfreisetzung aufweisen.

Hohe Auslöseraten von Fluorid sind in Glasionomerzementen relativ einfach zu verwirklichen. Das aufgrund einer Reaktion von fluoridhaltigem Glas und einer wässrigen Polycarbonsäurelösung aushärtende Material bildet im festen Zustand einen hydratisierten Zement, der ideale Bedingungen für die Bereitstellung und Diffusion vön Fluoridionen aufweist.

Erheblich schwieriger zu erreichen ist die Auslösung von Fluorid aus Composite-Füllungsmaterialien, die auch Kunststoff-Füllungen genannt werden. Composite bestehen in der ausgehärteten Form im wesentlichen aus einer polymeren Kunststoff-Matrix auf Basis von (Meth)acrylat-Monomeren und einem hohen Anteil an Füllstoffen. Um die Verbindung zwischen Kunststoff-Matrix und Füllstoff vor dem schädlichen Einfluß von Wasser (Hydrolyse) zu schützen, sind Composite in der Regel unpolar und hydrophob formuliert.

Im Gegensatz zu der wasserhaltigen, hydrophilen Matrix von Glasionomerzementen behindert die hydrophobe Matrix von Compositen die Auslösung und Diffusion von Fluoridionen jedoch. Weiterhin wird das Einbringen von fluoridhaltigen Zusatzstoffen in Composite durch die Unpolarität und Hydrophobie dieser Systeme erschwert. Sehr anschaulich wird diese Problematik in B.F. Zimmermann et al., J. Dent. Res. 63 (1984) 689-692 beschrieben: "A major problem with incorporation of highly polar inorganic fluoride salts into low polarity polymer resins is phase separation and, consequently, loss of mechanical integrity...".

Die US-A-4 515 910 und US-A-4 572 920 (Rawls et al.) beschreiben Polymere, hergestellt durch die Copolymerisation von fluoridhaltigen Monomeren mit (Meth)acrylaten.

Die US-A-5 037 638 (Hammer et al.) beschreibt dentale Composite, enthaltend als spezielles fluoridhaltiges einpolymerisierbares Monomer Morpholinoethyl-methacryloyl-hydrofluorid (MEM HF). Das Einbringen von organischen Fluoriden in Mengen, die eine glasionomerzementähnliche Fluoridionen-Auslösung bewirken, schwächt jedoch die Polymermatrix. Die hohen Anforderungen an die mechanische Stabilität von Kunststoff-Füllmassen können durch derartige Massen nicht erfüllt werden.

Die EP-B-0 238 025 (Gasser et al.) beschreibt röntgenopake polymerisierbare Dentalmassen, enthaltend Yttrium-Fluorid und/oder in Wasser schwerlösliche komplexe Schwermetall-Fluoride, wie z.B. Bariumhexafluorozirkonat. Diese Verbindungen dienen ausschließlich zur Erhöhung der Röntgenopazität der Dentalmassen.

K. Aleksieva et al. (CA 103(6): 42608e) beschreiben Prothesenkunststoffe auf Basis von Polymethylmethacrylat, die Natriumfluorid oder Kaliumhexafluorotitanat enthalten. Sie fanden, daß Kaliumhexafluorotitanat im Gegensatz zu Natriumfluorid die Zugfestigkeit des Polymers schwächt. Sie kommen zu der Schlußfolgerung, daß das Kaliumhexafluorotitanat-haltige Material weniger gut zur Herstellung von dentalen Prothesen geeignet ist als das Natriumfluorid-haltige. Zudem ist Natriumfluorid bekanntermaßen toxisch.

Aufgabe der vorliegenden Erfindung ist es, ein dentales Composite-Material bereitzustellen, das eine den Glasionomerzementen vergleichbare Fluoridionenauslösung aufweist, und das zu dauerhaften Füllungen mit guter mechanischer Stabilität führt.

Gelöst wird diese Aufgabe durch polymerisierbare Dentalmassen, enthaltend:
(a) ein oder mehrere ethylenisch ungesättigte polymerisierbare Monomere auf Basis di- oder mehrfunktioneller (Meth)acrylate;
(b) Initiatoren und gegebenenfalls Aktivatoren;
(c) übliche Füllstoffe, sowie gegebenenfalls Pigmente, Thixotropiehilfsmittel, Weichmacher und weitere Hilfsmittel;
   die dadurch gekennzeichnet sind, daß sie zusätzlich
(d) ein oder mehrere ausreichend wasserlösliche anorganische komplexe Fluoride der allgemeinen Formel

   AₙMFₘ

   enthalten, worin
   A ein einwertiges Kation, M ein Element der III-V Hauptoder II-V Nebengruppe, n eine ganze Zahl von 1 bis 3 und m eine ganze Zahl von 3 bis 6 bedeuten, wobei MFₘ ausgewählt ist aus der Gruppe SiF₆²⁻, TiF₆²⁻, ZrF₆²⁻, AlF₆³⁻, ZnF₃⁻ und PF₆⁻ . Besonders bevorzugt ist ZnF₃⁻.
   A ist vorzugsweise ein Alkalimetallion oder NR₄⁺ mit R = C₁-C₁₈-Alkyl, Phenyl oder substituiertes Phenyl.

Die Substituenten für Phenyl werden vorzugsweise ausgewählt aus der Gruppe bestehend aus C₁-C₆-Alkylgruppen, C₁-C₆-Alkylgruppen, welche durch Halogenide oder stickstoff- oder sauerstoffhaltige Reste substituiert sind, Halogenide, C₁-C₆-Oxyalkylgruppen und C₁-C₆-Azaalkylgruppen.

A ist besonders bevorzugt ein Natrium- oder Kaliumion und R bedeutet besonders bevorzugt C₁-C₆-Alkyl.

Das komplexe Fluorid ist in den Massen vorzugsweise zu 2 bis 25 Gew.-%, in noch bevorzugterer Weise zu 5 bis 15 Gew.-% und insbesondere zu 5 bis 10 Gew.-%, bezogen auf das Gesamtgewicht der Masse (Komponenten (a) bis (d)) enthalten.

Die Initiatoren und gegebenenfalls Aktivatoren gemäß Komponente (b) machen 0,01 bis 10 Gew.-%, insbesondere 0,1 bis 5 Gew.-% der Gesamtmasse (Komponenten (a) bis (d)) aus, und die Füllstoffe sind in den erfindungsgemäßen Massen in einem Anteil von vorzugsweise 40 bis 85 Gew.-% und insbesondere 50 bis 80 Gew.-%, bezogen auf die Gesamtmasse (Komponenten (a) bis (d)) enthalten.

Die erfindungsgemäß als Bestandteil (a) einzusetzenden, mindestens bifunktionellen Acrylsäure- und/oder Methacrylsäureester können monomere und polymere Acrylate und Methacrylate beinhalten. Vorteilhaft verwendet werden können beispielsweise die langkettigen Monomeren der US-A-3 066 112 auf der Basis von Bisphenol A und Glycidylmethacrylat oder deren durch Addition von Isocyanaten entstandene Derivate. Geeignet sind auch Verbindungen des Typs Bisphenol-A-diethyloxy(meth)-acrylat und Bisphenol-A-dipropyloxy(meth)acrylat. Weiterhin Verwendung finden können die oligo-ethoxylierten und oligopropoxylierten Bisphenol-A-diacryl- und -dimethacrylsäureester.

Gut geeignet sind weiter die Acrylsäure- und Methacrylsäureester mindestens bifunktioneller aliphatischer Alkohole, beispielsweise Triethylenglykol-di(meth)-acrylat, Ethylenglykol-di(meth)-acrylat, Hexandiol-di(meth)-acrylat und Trimethylolpropan-tri(meth)-acrylat.

Besonders geeignet sind auch die in der DE-C-2 816 823 genannten Diacryl- und Dimethacrylsäureester des Bis(hydroxymethyl)-tricyclo*[*5.2.1.0^{2,6}*]*-decans und die Diacryl- und Dimethacrylsäureester der mit 1 bis 3 Ethylenoxid- und/oder Propylenoxideinheiten verlängerten Verbindungen des Bis(hydroxymethyl)-tricyclo*[*5.2.1.0.^{2,6}*]*-decans.

Gut geeignete Monomere sind auch die in der EP-A-0 235 826 beschriebenen Methacrylsäureester, z.B. Triglykolsäure-bis *[*3(4)-methacryloxymethyl-8 (9)-tricyclo*[*5.2.1.0^{2,6}*]*-decylmethylester*]*.

Selbstverständlich können auch Gemische aus Monomeren und/oder aus hieraus hergestellten ungesättigten Polymeren verwendet werden. Die eingesetzte Menge an Komponente (a) beträgt vorzugsweise 10 bis 55 gew.-%, insbesondere 14 bis 44 Gew.-%, bezogen auf die gesamtmasse von (a) + (b) + (c) + (d).

Als Bestandteil (b) eignen sich Initiatorsysteme, die die radikalische Polymerisation der mindestens bifunktionellen Monomeren bewirken, z.B. Photoinitiatoren oder sogenannte Redoxinitiatorsysteme.

Als Photoinitiatoren eignen sich beispielsweise α-Diketone, wie Campherchinon, in Verbindung mit sekundären und tertiären Aminen, oder Mono- und Bisacylphosphinoxide, wie 2,4,6-Trimethylbenzoyldiphenyl-phospinoxid und Bis-(2,6-dichlorbenzoyl)-4-n-propylphenyl-phosphinoxid. Es eignen sich aber auch andere Verbindungen dieses Typs, wie sie in den europäischen Patentveröffentlichungsschriften EP-A-0 073 413, EP-A-0 007 508, EP-A-0 047 902, EP-A-0 057 474 und EP-A-0 184 095 beschrieben sind.

Die Konzentration der Photoinitiatoren beträgt in besonders bevorzugter Weise 0,1 bis 3 Gew.-%, und insbesondere 0,1 bis 2 Gew.-%, bezogen auf die Gesamtmasse von (a) + (b) + (c) + (d).

Als Redoxinitiatorsysteme eignen sich organische Peroxidverbindungen zusammen mit sogenannten Aktivatoren. Als organische Peroxidverbindungen kommen dabei insbesondere Verbindungen wie Lauroylperoxid, Benzoylperoxid sowie p-Chlorbenzoylperoxid und p-Methylbenzoylperoxid in Betracht.

Als Aktivatoren eignen sich beispielsweise tertiäre aromatische Amine, wie die aus der US-A-3 541 068 bekannten N,N-Bis-(hydroxyalkyl)-3,5-xylidine sowie die aus der DE-A-2 658 530 bekannten N,N-Bis-(hydroxyalkyl)-3,5-di-t-butylaniline, insbesondere N,N-Bis-(β-oxybutyl)-3,5-di-t-butylanilin sowie N,N-Bis-(hydroxyalkyl)-3,4,5-trimethylaniline.

Gut geeignete Aktivatoren sind auch die in der DE-B-1 495 520 beschriebenen Barbitursäuren und Barbitursäurederivate sowie die in der EP-A-0 059 451 beschriebenen Malonylsulfamide. Bevorzugte Malonylsulfamide sind 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Dioctyl-4-isobutylmalonylsulfamid.

Zur weiteren Beschleunigung wird die Polymerisation hierbei vorzugsweise in Gegenwart von Schwermetallverbindungen und ionogenem Halogen oder Pseudohalogen durchgeführt. Als Schwermetall ist Kupfer, als Halogenid das Chloridion besonders geeignet. Das Schwermetall wird geeigneterweise in Form löslicher organischer Verbindungen eingesetzt. Ebenso werden die Halogenid- und Pseudohalogenidionen geeigneterweise in Form von löslichen Salzen eingesetzt, beispielsweise genannt seien die löslichen Aminhydrochloride sowie quartäre Ammoniumchloridverbindungen.

Wenn die erfindungsgemäßen polymerisierbaren Dentalmassen als (b) ein Redoxinitiatorsystem aus organischem Peroxid und Aktivator enthalten, so sind vorzugsweise Peroxid und Aktivator in räumlich voneinander getrennten Teilen der erfindungsgemäßen Dentalmasse vorhanden, die erst unmittelbar vor der Anwendung der erfindungsgemäßen Dentalmasse homogen miteinander vermischt werden. Enthält die erfindungsgemäße Dentalmasse als (b) nebeneinander organisches Peroxid, Kupferverbindung, Halogenid und Malonylsulfamid, so ist es insbesondere sinnvoll, daß organisches Peroxid, Malonylsulfamid und die Kombination Kupferverbindung/Halogenid in drei räumlich voneinander getrennten Bestandteilen vorliegen. Beispielsweise können organisches Peroxid, polymerisierbare Monomere sowie Füllstoffe zu einer Paste verknetet sein und die anderen Komponenten in oben beschriebener Weise jeweils mit einer geringen Menge an Füllstoffen oder insbesondere Thixotropie-Hilfsmitteln, wie silanisierter Kieselsäure, und einem Weichmacher, beispeilsweisen Phthalat, zu zwei separaten Pasten verknetet sein. Andererseits können die polymerisierbaren Monomeren auch zusammen mit Kupferverbindung/Halogenid und Füllern vorliegen. Der Bestandteil (d). kann, falls die erfindungsgemäße Dentalmasse in räumlich voneinander getrennten Bestandteilen vorliegt, in jedem dieser Bestandteile vorhanden sein.

Außer den mindestens bifunktionellen Acrylsäure- und Methacrylsäureester (a) und dem Initiatorsystem (b) sind bis zu 85 Gew.-%, bezogen auf die Gesamtmasse von (a) + (b) + (c) + (d), organische und/oder anorganische Füllstoffe, Pigmente, Farbstoffe, Thixotropie-Hilfsmittel, Weichmacher und andere Hilfsstoffe enthalten.

Anorganische Füllstoffe können beispielsweise Quarz, gemahlene Gläser, nicht wasserlösliche Fluoride, wie CaF₂ oder SrF₂, Kieselgele sowie Kieselsäure, insbesondere pyrogene Kieselsäure oder deren Granulate, sein. Sie sind vorzugsweise in einer Konzentration von 40 bis 85 Gew.-%, ganz besonders bevorzugt von 50 bis 80 Gew.-%, bezogen auf die Gesamtmasse von (a) + (b) + (c) + (d), in den Dentalmassen enthalten. Zum besseren Einbau in die Polymermatrix kann es von Vorteil sein, die Füllstoffe sowie gegebenenfalls röntgenopake Zusatzstoffe zu hydrophobieren. In einer bevorzugten Ausführungsform sind sämtliche eingesetzten anorganischen Füllstoffe silanisiert, vorzugsweise mit Trimethoxymethacryloxypropylsilan. Die Menge des eingesetzten Silans beträgt üblicherweise 0,5 bis 10 Gew.-%, bezogen auf anorganische Füllstoffe, bevorzugt 1 bis 6 %, ganz besonders bevorzugt 2 bis 5 Gew.-%, bezogen auf anorganische Füllstoffe. Ubliche Hydrophobierungsmittel sind Silane, beispielsweise Trimethoxymethacryloxypropylsilan. Die maximale mittlere Korngröße der anorganischen Füllstoffe beträgt vorzugsweise 15 µm, insbesondere 8 µm. Ganz besonders bevorzugt werden Füllstoffe mit einer mittleren Korngröße von <3 µm eingesetzt.

Als Füllstoffe eignen sich auch bereits fertig pigmentierte Polymethylmethacrylatperlen oder andere pulverisierte organische Polymerisate. Zur Erhöhung der Flexibilität der Massen kann es auch vorteilhaft sein, lösliche organische Polymere einzustzen. Geeignet sind z.B. Polyvinylacetat sowie die Copolymeren auf Basis Vinylchlorid/Vinylacetat, Vinylchlorid/Vinylisobutylether und Vinylacetat/Maleinsäuredibutylether. Gut geeignet als zusätzliche Weichmacher sind beispielsweise Dibutyl-, Dioctyl- und Dinonylphthalate.

Die erfindungsgemäßen Massen können u.a. Einsatz finden als Dauerfüllungsmassen, Versiegelungsmassen, Zemente oder als Massen für provisorische Füllungen oder Kronen und Brücken.

Die erfindungsgemäßen polymerisierbaren fluoridabgebenden Dentalmassen verfügen über gute mechanische Werte (Druckund Biegefestigkeit), gute Langzeitstabilität des ausgehärteten Materials und ausgezeichnete Ästhetik. Ihre Fluoridionenauslösung ist der eines handelsüblichen Glasiönomerzements vergleichbar.

### Beispiele

### Serie 1: Herstellung und Testung von herkömmlichen und fluoridabgebenden Composite-Zahnfüllmassen mit zahnähnlicher Einfärbung.

### Herstellung einer Vormischung

Aus 70 Gewichtsteilen Bis-Acryloxymethyltricyclo (2.5.1.0^{2,6})-decan und 30 Gewichtsteilen 2,2-Bis-4-(3-methacryloxypropoxy)-phenylpropan (modifiziertes Bis-GMA), 24 Gewichtsteilen silanisierter pyrogener Kieselsäure, 0,3 Gewichtsteilen Campherchinon, 3 Gewichtsteilen N-Dimethylaminoethylmethacrylat und 110 Gewichtsteilen YF₃ wird eine Vormischung geknetet.

### Kontrollbeispiel

3,9 g der Vormischung und 6,1 g silanisierter zahnähnlich pigmentierter Quarz (Kornobergrenze ca. 3 µm, mittlere Korngröße ca. 1,5 µm) werden zu einer herkömmlichen Zahnfüllmasse mit einheitlich pastöser Konsistenz verknetet.

### Beispiel und Vergleichsbeispiel 1

3,9 g der Vormischung werden für die erfindungsgemäße Paste mit 1,6 g pulverisiertem Kaliumhexafluorotitanat bzw. für die Vergleichspaste mit 1,6 g pulverisiertem Natriumfluorid und jeweils 4,5 g silanisiertem, zahnähnlich pigmentierten Quarz (mittlere Korngröße 1,5 µm) zu einer Zahnfüllmasse mit einheitlich pastöser Konsistenz verarbeitet.

### Vergleichsbeispiel 2

3,9 g der Vormischung werden mit 0,4 g pulverisiertem Natriumfluorid und 5,7 g silanisiertem, zahnähnlich pigmentierten Quarz (mittlere Korngröße 1,5 µm) zu einer Zahnfüllmasse mit einheitlich pastöser Konsistenz verarbeitet.

Die zahnfarbenen Pasten des Kontrollbeispiels, des erfindungsgemäßen Beispiels und der Vergleichsbeispiele 1 bis 2 wurden nach den unterhalb der folgenden Tabelle 1 angegebenen Vorschriften zu Testkörpern geformt und ausgehärtet. Anschließend wurden die Druckfestigkeit, die Biegefestigkeit und die Opazität der Testkörper bestimmt. Die Ergebnisse der Untersuchungen sind in Tabelle 1 unten zusammengefaßt.

**TABELLE 1**

| Mechanische Eigenschaften der ausgehärteten zahnfarbenen Composite (Serie 1: Kontrollbeispiel, Beispiel und Vergleichsbeispiele 1 und 2) | | | | |
|---|---|---|---|---|
| | Kontrollbeispiel | Beispiel | Vergleichsbeispiel 1 | Vergleichsbeispiel 2 |
| Druckfestigkeit1) *[*MPa*]* | 420 | 410 | 340 | 360 |
| Biegefestigkeit 2) *[*MPa*]* | 110 | 102 | 87 | 90 |
| Opazität 3) *[*%*]* | 92,0 | 93,3 | 99,8 | 98,0 |

| | | | | |
|---|---|---|---|---|
| 1) Druckfestigkeit gemessen nach ISO 9917 | | | | |
| 2) Biegefestigkeit gemessen nach ISO 4049 | | | | |
| 3) Opazität gemessen mit einem Cielab-Farbmeßgerät (Prüfkörperscheibendurchmesser 2 cm, Höhe 3,5 mm) | | | | |

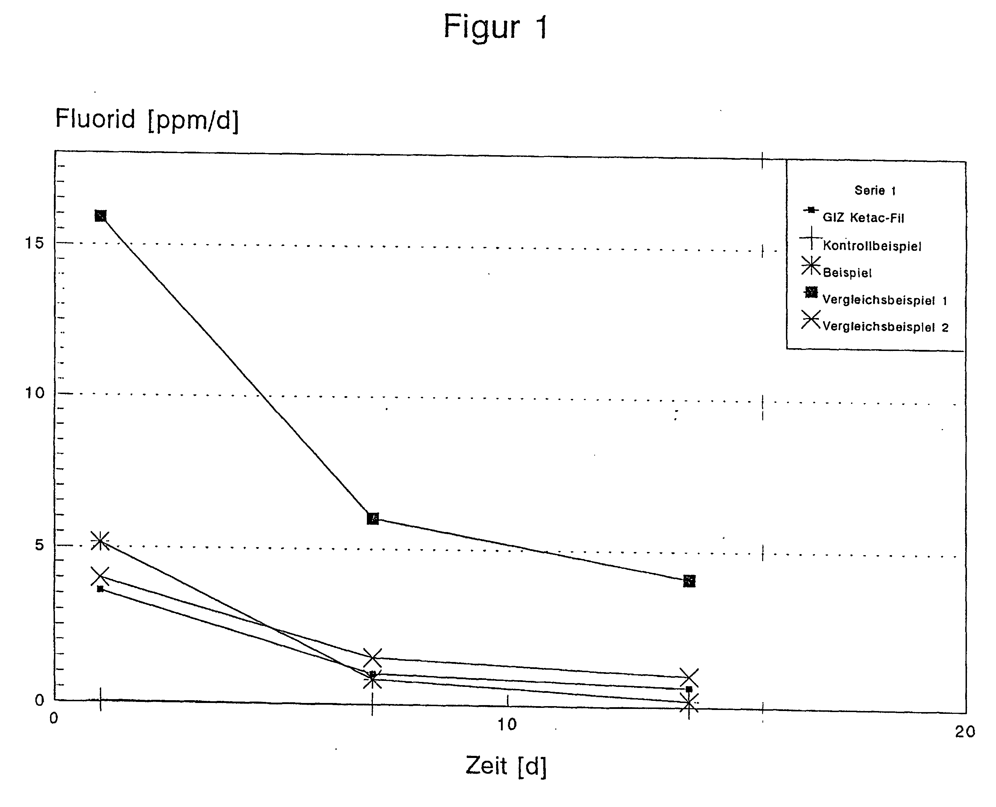

In weiteren Untersuchungen wurde die Fluoridionenabgabe der ausgehärteten zahnfarbenen Coposite des Kontrollbeispiels, des erfindungsgemäßen Beispiels und der Vergleichsbeispiele 1 und 2 sowie eines handelsüblichen Glasionomerzements ermittelt. Die Ergebnisse sind in der nachfolgenden Tabelle 2 zusammengefaßt und in der Figur 1 graphisch dargestellt. Die Fluoridionenabgabe ist überraschend hoch und mit der des handelsüblichen Glasionomerzements vergleichbar.

**TABELLE 2**

| Fluoridabgabe¹⁾der Composite der Serie 1 (ppm F/d) | | | |
|---|---|---|---|
| | 1 Tag | 7 Tage | 14 Tage |
| GIZ Ketac-Fil | 3,60 | 1,00 | 0,62 |
| Kontrollbeispiel | 0,05 | 0,01 | 0,01 |
| Beispiel | 5,16 | 0,82 | 0,19 |
| Vergleichsbeispiel 1 | 15,90 | 6,00 | 4,10 |
| Vergleichsbeispiel 2 | 4,00 | 1,50 | 1,00 |

| | | | |
|---|---|---|---|
| ¹⁾ Zwei Probekörper (Ø 15 mm, h 1,5 mm) werden frei hängend in 50 ml destilliertem Wasser bei 36° gelagert und nach dem jeweiligen Zeitraum die Fluoridkonzentration mittels ionensensitiver Elektrode gemessen. Nach jeder Messung Wechsel des Wassers. Die Angaben beziehen sich auf die tägliche Abgabe. | | | |

### Serie 2: Herstellung und Testung von herkömmlichen und fluoridabgebenden Composite-Zahnfüllmassen ohne Einfärbung

### Herstellung einer Vormischung

Aus 70 Gewichtsteilen Bis-Acryloxymethyltricyclo(2.5.1.0^{2.6})-decan und 30 Gewichtsteilen 2,2-Bis-4-(3-methacryloxypropoxy)-phenylpropan, 24 Gewichtsteilen silanisierter pyrogener Kieselsäure, 0,3 Gewichtsteilen Campherchinon, 3 Gewichtsteilen N-Dimethylaminoethylmethacrylat und 110 Gewichtsteilen YF₃ wird eine Vormischung geknetet.

### Herstellung der Composite-Pasten

Die in der Tabelle 3 angegebene Menge an Vormischung wird jeweils mit den angegbenen Mengen an fluoridabgebendem Füllstoff und nicht-pigmentiertem Quarz zu Zahnfüllmassen vergleichbarer pastöser Konsistenz verarbeitet. Die Konsistenz wird anhand der Viskosität kontrolliert.

### Eigenschaften der Composite

Tabelle 3 gibt die Zusammensetzungen der Beispiele 1 und 2 und des Kontrollbeispiels bzw. der Vergleichsbeispiele 1 bis 4 der Serie 2 wieder. Bei vergleichbarem Gehalt an fluoridabgebendem Füllstoff und vergleichbarer Viskosität (= Verarbeitungseigenschaften) weisen die erfindungsgemäßen Composite einen erhöhten Gesamtfüllstoffgehalt auf. Hierdurch wird der auf die Kunststoffmatrix zurückgehende Polymerisationsschrumpf gegenüber niedriger gefüllten Systemen vermindert (vgl. Tabelle 4). Dies kann der Tendenz zu Randspaltbildungen entgegenwirken.

Tabelle 4 zeigt, daß die erfindungsgemäßen Composite neben ausgezeichneten mechanischen Werten im nicht eingefärbten Zustand auch gute Ästhetik (= niedrige Opazität) aufweisen. Gegenüber den Massen mit Natriumfluorid (Vergleichsbeispiele 2 und 3), die stumpf und leblos wirken, weisen die erfindungsgemäßen Massen zum Teil zahnähnliche Transparenz und Ästhetik auf.

Insbesondere der Vergleich der K₂TiF₆- mit den NaF-haltigen Massen ist überraschend K. Aleksieva et al. berichten, daß NaF als fluoridabgebender Bestandteil die mechanischen Werte von PMMA-Prothesenkunststoffen deutlich weniger schwächt als K₂TiF₆. Für die Composite-Massen auf Basis difunktioneller (Meth)acrylate wird unerwarteterweise das Gegenteil gefunden.

Tabelle 5 zeigt, daß in einem Hydrolysestabilitätstest (10 h Einlagerung in Wasser bei 100°C) die Oberflächenhärte der erfindungsgemäßen Massen deutlich stärker ansteigt (sogenannter Nachvergütungseffekt) als bei den Vergleichsbeispielen.

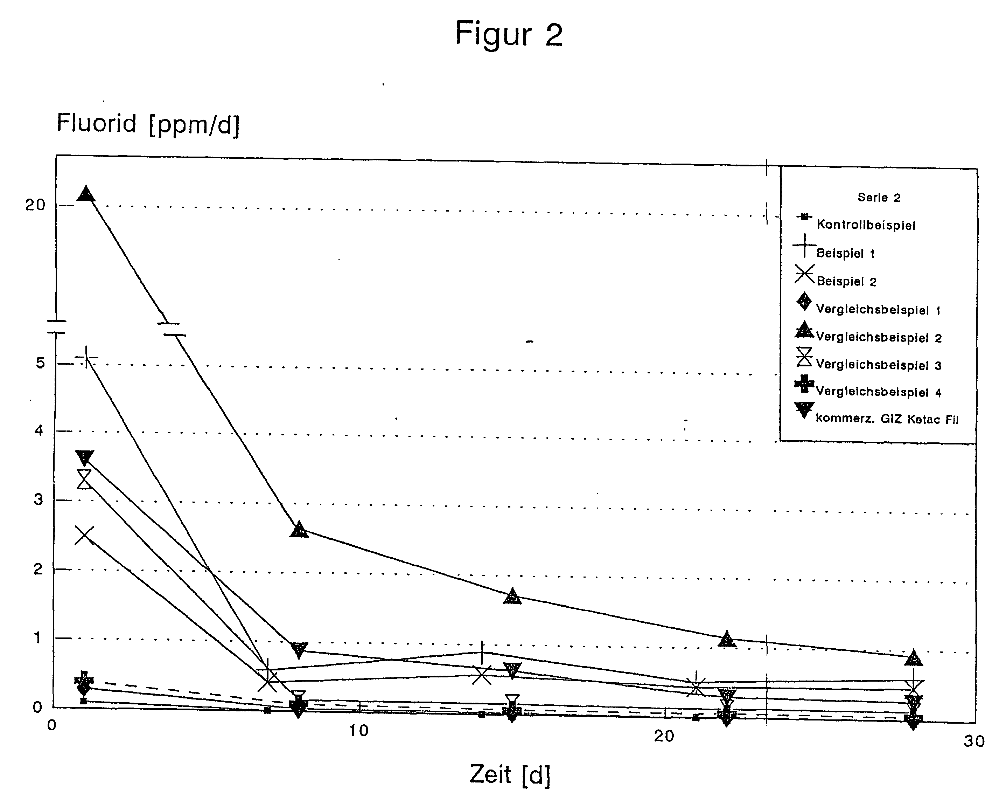

Tabelle 6 gibt eine Übersicht über die Fluoridfreisetzung aus den Compositen. Figur 2 stellt diese graphisch dar. Mit den erfindungsgemäßen Compositen werden Auslöseraten erzielt, die vergleichbar mit kommerziell erhältlichen Glasionomerzementen (z.B. Ketac Fil, Fa. ESPE, Seefeld) sind. Mit NaF als fluoridabgebenden Füllstoff (siehe Vergleichsbeispiel 2) sind zwar prinzipiell noch höhere Auslösungsraten möglich, aufgrund der schlechten mechanischen Werte und der mangelhaften Ästhetik (vgl. Tabelle 3 und 4) sind NaF-haltige Massen den erfindungsgemäßen Copositen aber deutlich unterlegen.

Die erfindungsgemäßen Rezepturen erlauben also die Formulierung von Compositen als zahnärztliche Werkstoffe, die folgende Anforderungen in guter Weise erfüllen:
- Fluoridabgabe vergleichbar den Glasionomerzementen
- gute mechanische Werte
- ausgezeichnete Ästhetik
- hohe Stabilität in wässrigem Milieu
und die damit eine deutliche Verbesserung gegenüber dem Stand der Technik darstellen.

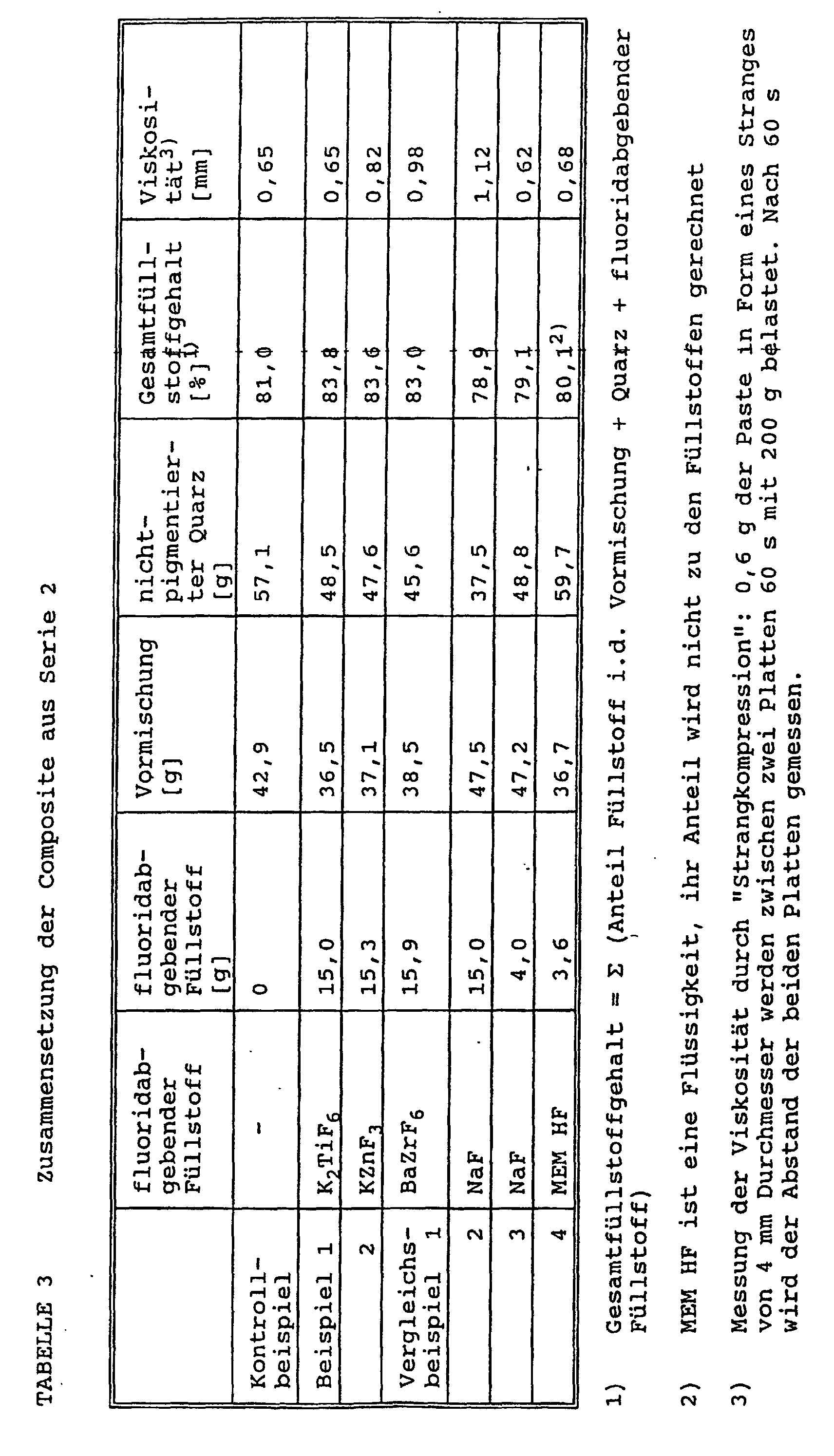

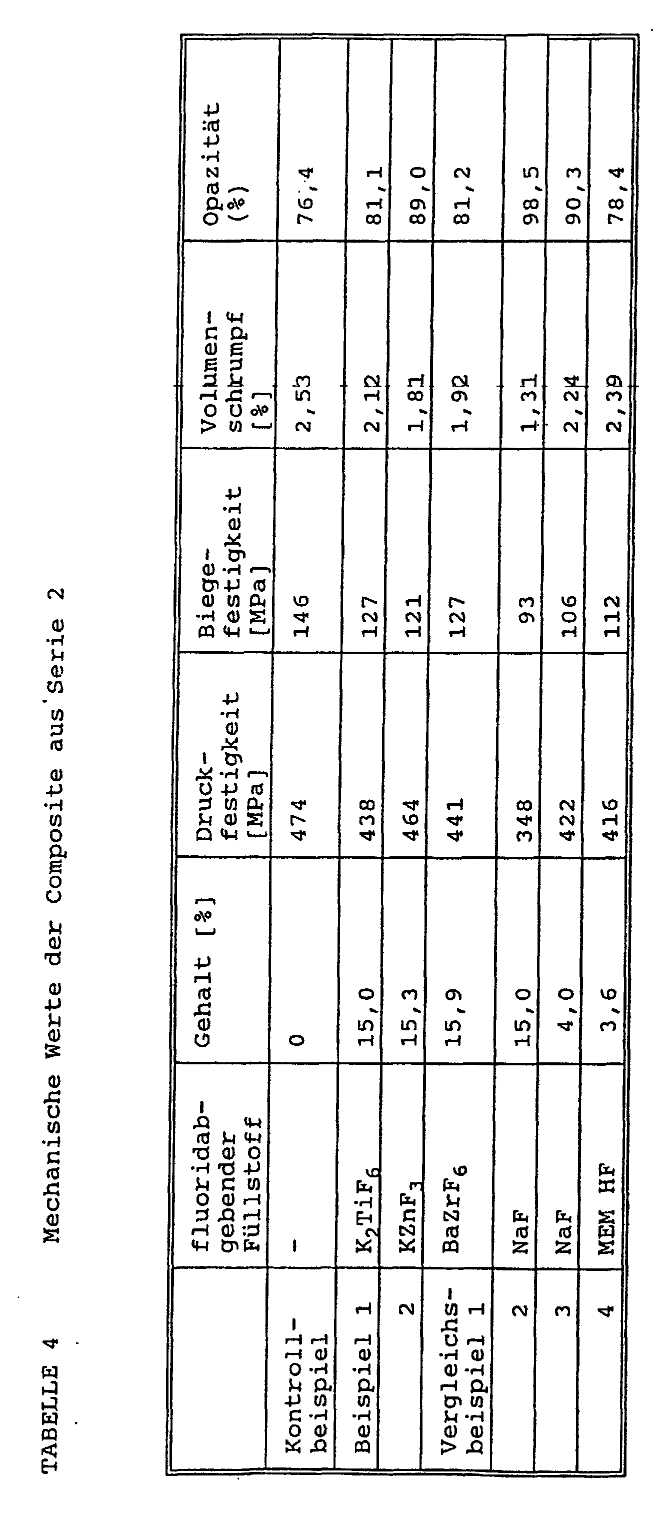

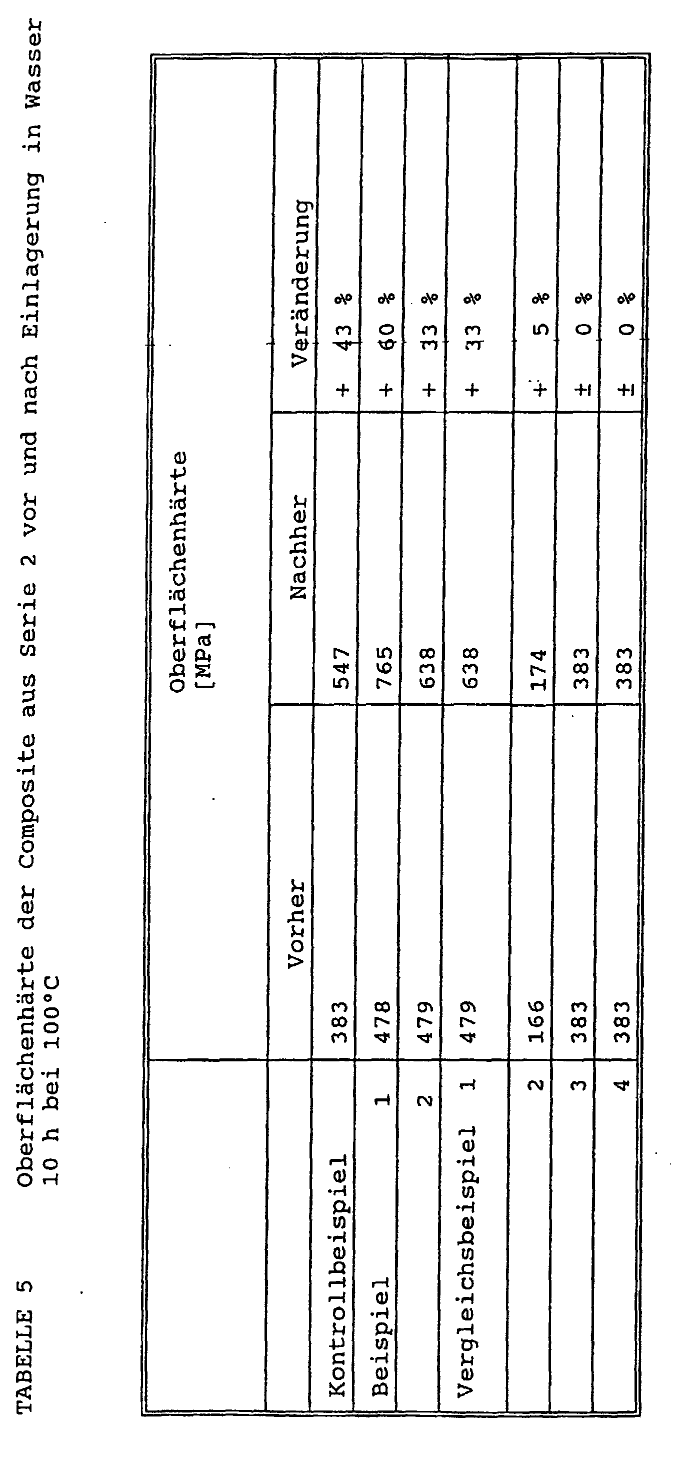

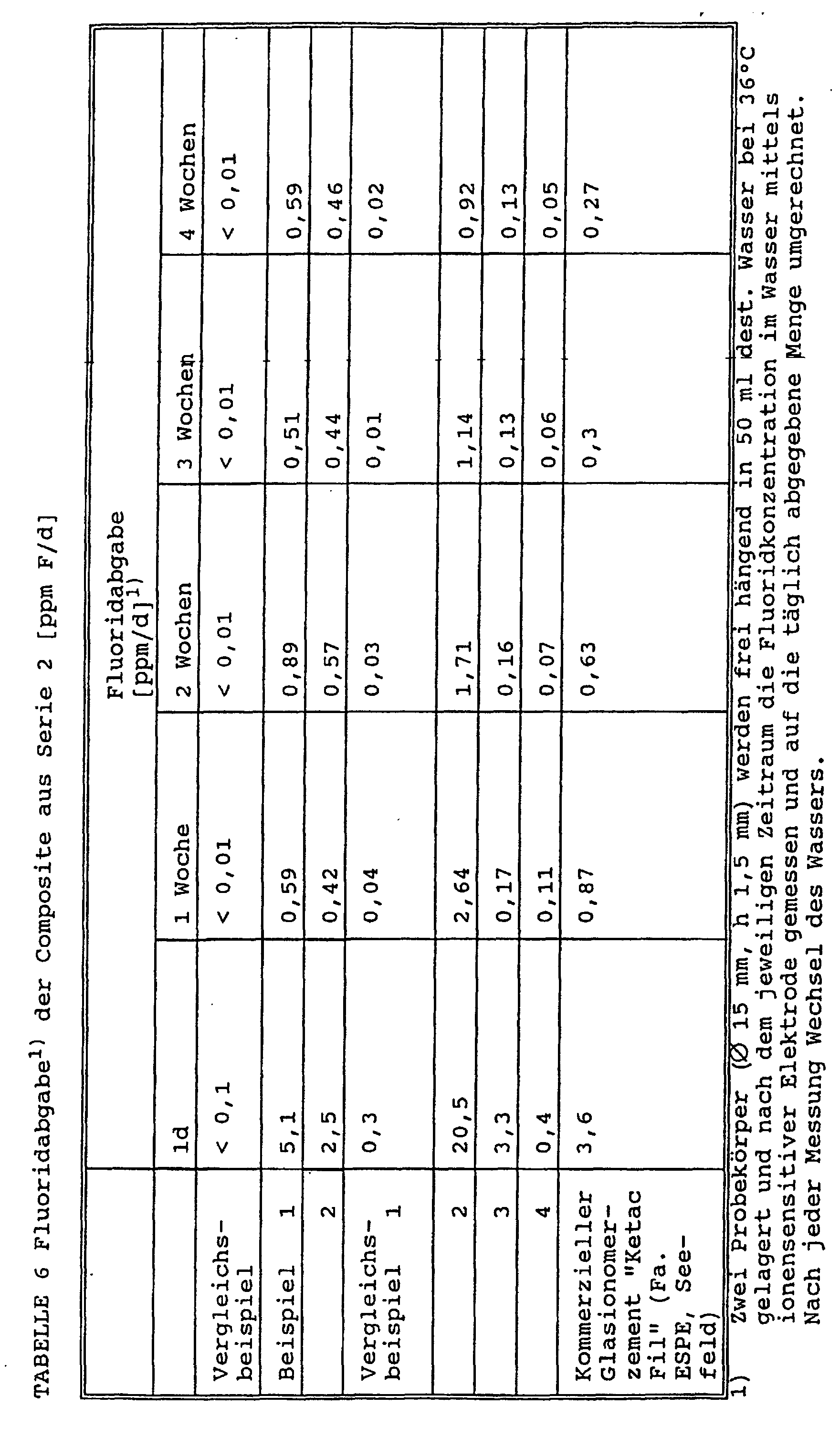

## Patentansprüche

1. Polymerisierbare Dentalmasse enthaltend:
(a) ein oder mehrere ethylenisch ungesättigte polymerisierbare Monomere auf Basis di- oder mehrfunktioneller (Meth)acrylate;
(b) Initiatoren und gegebenenfalls Aktivatoren;
(c) übliche Füllstoffe, sowie gegebenenfalls Pigmente, Thixotropiehilfsmittel, Weichmacher und weitere Hilfsmittel,
**dadurch gekennzeichnet, daß** sie zusätzlich
(d) ein oder mehrere ausreichend wasserlösliche anorganische komplexe Fluoride der allgemeinen Formel
AₙMFₘ
enthält, worin
A ein einwertiges Kation, M ein Element der III-V Haupt- oder II-V Nebengruppe, n eine ganze Zahl von 1 bis 3 und m eine ganze Zahl von 3 bis 6 bedeuten, wobei die Gruppe MFₘ ausgewählt ist unter SiF₆²⁻, TiF₆²⁻, ZrF₆²⁻, AlF₆³⁻, ZnF₃⁻ und PF₆⁻.
(es folgen die Patentansprüche 1 bis 6 gemäß Hauptantrag)

2. Dentalmasse nach Anspruch 1, **dadurch gekennzeichnet, daß** A ein Alkalimetallion, vorzugsweise ein Natriumoder ein Kaliumion, oder NR₄⁺ mit R = C₁-C₁₈-Alkyl, Phenyl oder substituiertes Phenyl bedeutet.

3. Dentalmasse nach Anspruch 2, **dadurch gekennzeichnet, daß** R substituiertes Phenyl bedeutet und die Substituenten für Phenyl ausgewählt sind aus der Gruppe bestehend aus C₁-C₆-Alkylgruppen, C₁-C₆-Alkylgruppen, welche durch Halogenide oder stickstoff- oder sauerstoffhaltige Reste substituiert sind, Halogenide, C₁-C₆-Oxyalkylgruppen und C₁-C₆-Azaalkylgruppen.

4. Dentalmasse nach Anspruch 2, **dadurch gekennzeichnet, daß** R einen C₁-C₆-Alkylrest bedeutet.

5. Dentalmasse nach den Ansprüchen 1 bis 4, **dadurch gekennzeichnet, daß** sie enthält
10 bis 55 Gew.-% Komponente (a),
0,01 bis 10 Gew.-% Komponente (b),
40 bis 85 Gew.-% Komponente (c) und
2 bis 25 Gew.-% Komponente (d).

6. Dentalmasse nach den Ansprüchen 1 bis 5, **dadurch gekennzeichnet, daß** sie enthält
14 bis 44 Gew.-% Komponente (a),
0,1 bis 5 Gew.-% Komponente (b),
50 bis 80 Gew.-% Komponente (c) und
5 bis 15 Gew.-%, insbesondere 5 bis 10 Gew.-% Komponente (d).

7. Verwendung von einem oder mehreren ausreichend wasserlöslichen anorganischen komplexen Fluoriden der allgemeinen Formel
AₙMFₘ,
worin A ein einwertiges Kation, M ein Elementder III-V Haupt- oder II-V Nebengruppe, n eine ganze Zahl von 1 bis 3 und m eine ganze Zahl von 3 bis 6 bedeuten, wobei die Gruppe MFₘ ausgewählt ist unter SiF₆²⁻, TiF₆²⁻, ZrF₆²⁻, AlF₆³⁻, ZnF₃⁻ und PF₆⁻, zur Herstellung von polymerisierbaren, fluoridabgebenden Dentalmassen, die neben dem anorganischen komplexen Fluorid enthalten:
(a) ein oder mehrere ethylenisch ungesättigte polymerisierbare Monomere auf der Basis di- oder mehrfunktioneller (Meth)acrylate;
(b) Initiatoren und gegebenenfalls Aktivatoren; und
(c) übliche Füllstoffe, sowie gegebenenfalls Pigmente, Thixotropiehilfsmittel, Weichmacher und weitere Hilfsmittel.

## Claims

1. Polymerizable dental material containing:
(a) one or more ethylenically unsaturated polymerizable monomers based on di- or multi-functional (meth)acrylates;
(b) initiators and optionally activators;
(c) usual fillers, and optionally pigments, thixotropic auxiliaries, plasticizers and other auxiliaries,
**characterized in that** it additionally contains
(d) one or more sufficiently water-soluble inorganic complex fluorides of general formula
AₙMFₘ
where
A is a monovalent cation, M is an element of the III-V main group or II-V sub-group, n is an integer from 1 to 3 and m is an integer from 3 to 6, the group MFₘ being selected from SiF₆²⁻, TiF₆²⁻, ZrF₆²⁻, AlF₆³⁻, ZnF₃⁻ and PF₆⁻.

2. Dental material according to claim 1, **characterized in that** A is an alkali metal ion, preferably a sodium or a potassium ion, or NR₄⁺ with R = C₁-C₁₈ alkyl, phenyl or substituted phenyl.

3. Dental material according to claim 2, **characterized in that** R is substituted phenyl and the substituents for phenyl are selected from the group consisting of C₁-C₆ alkyl groups, C₁-C₆ alkyl groups which are substituted by halides or nitrogen or oxygen-containing radicals, halides, C₁-C₆ oxyalkyl groups and C₁-C₆ azaalkyl groups.

4. Dental material according to claim 2, **characterized in that** R is a C₁-C₆ alkyl radical.

5. Dental material according to claims 1 to 4, **characterized in that** it contains
10 to 55 wt.-% component (a),
0.01 to 10 wt.-% component (b)
40 to 85 wt.-% component (c) and
2 to 25 wt.-% component (d).

6. Dental material according to claims 1 to 5, **characterized in that** it contains
14 to 44 wt.-% component (a)
0.1 to 5 wt.-% component (b)
50 to 80 wt.-% component (c) and
5 to 15 wt.-%, in particular 5 to 10 wt.-% component (d).

7. Use of one or more sufficiently water-soluble inorganic complex fluorides of general formula
AₙMFₘ,
where A is a monovalent cation, M is an element of the III-V main group or II-V sub-group, n is an integer from 1 to 3 and m is an integer from 3 to 6, the group MFₘ being selected from SiF₆²⁻, TiF₆²⁻, ZrF₆²⁻, AlF₆³⁻, ZnF₃⁻ and PF₆⁻, for producing polymerizable, fluoride-releasing dental materials, which, in addition to the inorganic complex fluoride, contain:
(a) one or more ethylenically unsaturated polymerizable monomers based on di- or multi-functional (meth)acrylates;
(b) initiators and optionally activators; and
(c) usual fillers, and optionally pigments, thixotropic auxiliaries, plasticizers and other auxiliaries.

## Revendications

1. Composition dentaire polymérisable contenant :
(a) un ou plusieurs monomères polymérisables éthyléniquement insaturés à base de (méth)acrylates bi- ou polyfonctionnels ;
(b) des initiateurs et, le cas échéant, des activateurs ;
(c) des charges usuelles ainsi que, le cas échéant, des pigments, des auxiliaires de thixotropie, des plastifiants et d'autres auxiliaires,
**caractérisée en ce qu'**elle contient, en outre,
(d) un ou plusieurs fluorures complexes inorganiques suffisamment solubles dans l'eau, répondant à la formule générale
AₙMFₘ
dans laquelle
A signifie un cation monovalent, M signifie un élément du groupe principal III-V ou du groupe secondaire II-V, n signifie un nombre entier de 1 à 3, et m signifie un nombre entier de 3 à 6, le groupe MFₘ étant choisi parmi SiF₆²⁻, TiF₆²⁻, ZrF₆²⁻, AlF₆³⁻, ZnF₃⁻ et PF₆⁻.

2. Composition dentaire selon la revendication 1, **caractérisée en ce que** A signifie un ion de métal alcalin, de préférence un ion sodium ou potassium, ou NR₄⁺ avec R = alkyle en C₁-C₁₈, phényle ou phényle substitué.

3. Composition dentaire selon la revendication 2, **caractérisée en ce que** R signifie un groupe phényle, et les substituants pour le groupe phényle sont choisis dans l'ensemble constitué des groupes alkyle en C₁-C₆, des groupes alkyle en C₁-C₆ qui sont substitués par des halogénures ou par des radicaux contenant de l'azote ou de l'oxygène, des halogénures, des groupes oxyalkyle en C₁-C₆ et des groupes azaalkyle en C₁-C₆.

4. Composition dentaire selon la revendication 2, **caractérisée en ce que** R signifie un radical alkyle en C₁-C₆.

5. Composition dentaire selon les revendications 1 à 4,
**caractérisée en ce qu'**elle contient
de 10 à 55 % en poids de composant (a),
de 0,01 à 10 % en poids de composant (b),
de 40 à 85 % en poids de composant (c) et
de 2 à 25 % en poids de composant (d).

6. Composition dentaire selon les revendications 1 à 5,
**caractérisée en ce qu'**elle contient
de 14 à 44 % en poids de composant (a),
de 0,1 à 5 % en poids de composant (b),
de 50 à 80 % en poids de composant (c) et
de 5 à 15 % en poids, en particulier de 5 à 10 % en poids, de composant (d).

7. Utilisation d'un ou plusieurs fluorures complexes inorganiques suffisamment solubles dans l'eau, répondant à la formule générale
AₙMFₘ
dans laquelle A signifie un cation monovalent, M signifie un élément du groupe principal III-V ou du groupe secondaire II-V, n signifie un nombre entier de 1 à 3, et m signifie un nombre entier de 3 à 6, le groupe MFₘ étant choisi parmi SiF₆²⁻, TiF₆²⁻, ZrF₆²⁻, AlF₆³⁻, ZnF₃⁻ et PF₆⁻, pour la préparation de compositions dentaires polymérisables libérant du fluorure, qui, en plus du fluorure complexe inorganique, contiennent :
(a) un ou plusieurs monomères polymérisables éthyléniquement insaturés à base de (méth)acrylates bi- ou polyfonctionnels ;
(b) des initiateurs et, le cas échéant, des activateurs ; et
(c) des charges usuelles ainsi que, le cas échéant, des pigments, des auxiliaires de thixotropie, des plastifiants et d'autres auxiliaires.
